# EUROPÄISCHE PATENTSCHRIFT

(11) **EP 1 181 387 B1**
(45) Veröffentlichungstag und Bekanntmachung des Hinweises auf die Patenterteilung: **01.03.2006**
(21) Anmeldenummer: 00935150.3
(22) Anmeldetag: 30.05.2000
(51) Int. Cl.: C12Q 1/02

(54) **NEUES VERFAHREN ZUR UNTERSUCHUNG DER EIGNUNG EINES MATERIALS ALS PHARMAKON**
NOVEL METHOD FOR INVESTIGATING THE SUITABILITY OF A MATERIAL AS A MEDICAMENT
NOUVEAU PROCEDE POUR L'ETUDE DE LA CONVENANCE D'UNE SUBSTANCE EN TANT QUE MEDICAMENT

(30) Priorität: 31.05.1999 DE 19924929
(43) Veröffentlichungstag der Anmeldung: 27.02.2002
(73) Patentinhaber: Sanofi-Aventis Deutschland GmbH, 65929 Frankfurt am Main (DE)
(72) Erfinder: BAUMEISTER, Ralf, D-82194 Gröbenzell (DE); WITTENBURG, Nicole, D-82152 Planegg (DE)
(86) Internationale Anmeldenummer: PCT/EP2000/004950
(87) Internationale Veröffentlichungsnummer: WO 2000/073491

(56) Entgegenhaltungen:
- WO-A-00/34438
- WO-A-90/09096
- WITTENBURG NICOLE ET AL: "Thermal avoidance in Caenorhabditis elegans: An approach to the study of nociception." PROCEEDINGS OF THE NATIONAL ACADEMY OF SCIENCES OF THE UNITED STATES, Bd. 96, Nr. 18, 31. August 1999 (1999-08-31), Seiten 10477-10482, XP002152167 Aug. 31, 1999 ISSN: 0027-8424
- SAMOILOFF M R ET AL: "REGULATION OF NEMATODE BEHAVIOR BY PHYSICAL MEANS" EXPERIMENTAL PARASITOLOGY, Bd. 33, Nr. 2, 1973, Seiten 253-262, XP000964575 ISSN: 0014-4894
- AHRINGER J: "TURN TO THE WORM!" CURRENT OPINION IN GENETICS & DEVELOPMENT,XX,CURRENT BIOLOGY LTD, Bd. 7, Nr. 3, 1997, Seiten 410-415, XP000886904 ISSN: 0959-437X

## Beschreibung

Die vorliegende Erfindung betrifft ein Verfahren zur Untersuchung der Eignung eines Materials als Pharmakon für Säugetiere betreffend die Schmerzempfindung, das heißt insbesondere Menschen. Weiterhin betrifft die Erfindung einen Kit oder eine Ausrüstung, mit der sich ein solches Verfahren praktisch umsetzen läßt.

Die Entwicklung neuer Medikamente dauert durchschnittlich insgesamt 10 bis 12 Jahre. Ein Großteil dieser Zeit besteht zunächst darin, zielgerichtet ein Material zu finden, das sich für eine bestimmte Indikation eignen soll. Häufig geht die Zahl der zunächst einmal erwogenen Substanzen in die Hunderttausende. Somit besteht ein besonderes Bedürfnis nach einem einfachen und schnellen Testverfahren, nach dem sich die zu untersuchenden Substanzen grundsätzlich als geeignet oder ungeeignet zuordnen lassen. Es sind zur Zeit aber keine Verfahren bekannt, mit denen man in hoher Zuverlässigkeit eine Voraussage über eine mögliche Wirksamkeit beim Menschen (oder anderen Säugetieren) ermitteln kann. Selbstverständlich bieten sich hierzu Tiermodelle an, die aber hinsichtlich ihres Voraussagewertes entweder wenig zuverlässig und/oder sehr aufwendig sind. Konventionelle Tiermodelle werden zudem in zunehmendem Maße allgemein abgelehnt, da sie mit Qualen für die Tiere verbunden sind (beispielsweise Hunde und Affen). Andererseits wird aber von Tiermodellen regelmäßig gefordert, daß sie eine große Ähnlichkeit zu dem menschlichen Organismus aufweisen, damit eine gewisse Wahrscheinlichkeit für die aus den Tierversuchen gewonnen Kenntnisse bezüglich ihrer Übertragbarkeit auf den Menschen gewährleistet ist.

Es besteht ein besonderes Bedürfnis nach einem Tiermodell, das einen hohen Voraussagewert hat und entweder konventionelle Tiermodelle ersetzen oder doch zumindest den Umfang der Versuche mit höheren Lebewesen (wie Säugetieren) reduzieren kann. Dieses Bedürfnis besteht insbesondere im Zusammenhang mit der Suche nach Mitteln, die auf die menschliche Schmerzwahrnehmung Einfluß nehmen, wie z.B. Analgetika und Sedativa.

Aus der Schrift Samoiloff et al., "Regulation of Nematode Behavior by Physical Means", Experimental Parasitology 33, 253-262 (1973), ist bekannt, dass Einwirkung von Mikrowellen Strahlung, Lasermikrostrahleinwirkung und kurze Pulse elektrischen Stroms Änderungen des Schwimmverhaltens der freilebenden Fadenwürmer (Nematoden) Panagrellus Silusiae hervorrufen.

Aus der WO90/09096 ist ein Verfahren zur Wirkstoffsuche- und Klassifizierung bekannt, bei dem die Bioaktivität und Wirksamkeit zu untersuchender Wirkstoffe anhand des Reaktionsmusters einer Library von Nematoden auf diese Wirkstoffe untersucht und klassifiziert wird. Die Klassifizierung erfolgt dabei im Vergleich zum Reaktionsmuster von Wirkstoffen bekannter Wirkung.

Die vorliegende Erfindung geht nun auf die Entdeckung zurück, daß Nematoden (Fadenwürmer) auf einen örtlichen thermischen Reiz einen Rückzugsreflex aufweisen, der sich durch Wirkstoffe, die auf die menschliche Schmerzwahrnehmung einen Einfluß haben, beeinflussen läßt. Die vorliegende Erfindung beruht auf der Erkenntnis, daß bestimmte Fadenwürmer in definierter Weise auf lokal aufgebrachte thermische Reize reagieren und daß der Reflex auf diesen Reiz eine Vorhersage bezüglich der Wirkung eines bestimmten Materials auf die Schmerzempfindung von Menschen (einschließlich Säugetieren) zuläßt.

Die Erfindung besteht folglich in einem Verfahren zur Untersuchung der Eignung eines Materials als Pharmakon betreffend die Schmerzempfindung, wobei man zumindest einen Nematoden einsetzt, der dem zu untersuchenden Material ausgesetzt wurde, und dieser Nematode örtlich einem thermischen Reiz, vorzugsweise außerhalb eines Temperaturbereichs von 13-26°C, ausgesetzt wird. Infolge dieses Reizes zeigt der Nematode einen auffälligen Reflex, der in Abhängigkeit von dem eingesetzten Material von einem normalen Reflexverhalten (Normalverhalten) abweichen kann. Der thermische Reiz wird vorzugsweise auf einen Bereich von 10-100µm² begrenzt. Dieser Reiz kann mittels eines Lasers oder einer erwärmten Drahtspitze ausgelöst werden.

In der Regel wird es ausreichen, zur Feststellung einer Eignung bzw. Nicht-Bignung qualitativ die Abweichung von einem zuvor festgestellten Normal verhalten aufzuzeichnen. Da das Normalverhalten sehr ausgeprägt und signifikant ist (s. im einzelnen im Folgenden), wird eine Abweichung leicht festzustellen sein. Regelmäßig wird man größere Populationen der Nematoden untersuchen und eine qualitative Abweichung im wesentlichen als Prozentsatz ausdrücken können, nämlich als Prozentsatz der Tiere, die ein bestimmtes Verhalten nicht mehr zeigen, nachdem sie dem Material ausgesetzt wurden und die thermische Reizung ausgeführt wurde. Eine bestimmte Eignung kann sich aber auch darin zeigen, daß das Reflexverhalten verstärkt wird, insbesondere im Fall von Algetika. Im letzteren bietet es sich zusätzlich an, Mutanten der Nematoden einzusetzen, deren Reflexverhalten auf den thermischen Reiz nicht so ausgeprägt ist, die also dann infolge einer Verabreichung mit einer schmerzauslösenden Substanz ein verstärktes Reflexverhalten zeigen.

Die Anwendung des erfindungsgemäßen Verfahrens ist aber nicht auf ein solches Pharmakon beschränkt, das auf den Schmerz einen Einfluß nimmt. Eine Wirkung auf das Schmerzempfinden eines beispielsweise Menschen bedeutet nichts anderes, als daß bestimmte Rezeptoren durch eine bestimmte Verbindung angesprochen werden. Rezeptoren, die für bestimmte Wirkungen ursächlich sind, weisen jedoch häufig untereinander gewisse Ähnlichkeiten auf, so daß beispielsweise schmerzlindernde Substanzen auch bestimmte pharmkologische Wirkungen in anderen Indikationsbereichen haben. Solche Indikationsbereiche betreffen unter anderem antiinflammatorische, antipyretische, sedative, muskelrelaxierende und ähnliche Wirkungen. Unter Schmerz wird im Zusammenhang mit der vorliegenden Erfindung auch jegliche Art von Schmerz verstanden, wie beispielsweise Muskelschmerz oder Kopfschmerzen bzw. Migräne.

Bei Nematoden der Gattung Caenorhabditis und insbesondere bei *Caenorhabditis elegans* bzw. deren Mutanten wurde dieses Verhalten genauer untersucht. Bei diesem Fadenwurm beobachtet man im ausgewachsenen Zustand (aber nicht in der Diapause, den sogenannten Dauerlarven) einen ausgeprägten Reflex auf lokale thermische Reize. Durch diese thermischen Reize werden offensichtlich Nozizeptoren angeregt, die zu einem reflexartigen Rückzugsverhalten führen. Dieses Verhalten wird besonders auffällig ausgelöst, wenn man *C. elegans* im Kopf- oder Schwanzbereich mit entweder eiskalten Temperaturen oder einer lokalen Erwärmung von mindestens 28°C aussetzt. Es wurde festgestellt, daß dieses Verhalten nicht davon abhängt, bei welcher Temperatur die Nematoden aufgezogen wurden (Larvalentwicklung). Vielmehr zeigt *C. elegans* auf die lokale Einwirkung sogenannter noxischer, d.h. potentiell zellschädigender Hitze, ein Verhalten, das zwischen einem schnellen reflexivem Rückzug und einem langsamen Rückzug von der Applikationsstelle liegen kann.

Es wurde nun überraschend festgestellt, daß Nematoden, die bestimmten chemischen Substanzen mit einer pharmakologischen Wirkung beim Menschen ausgesetzt wurden, Abweichungen von dem zuvor beschriebenen Normalverhalten zeigen. Man kann die Nematoden in verschiedenster Weise den zu untersuchenden Materialien aussetzen. Beispielsweise ist es möglich, die Tiere in Petrischalen mit einem Agar zu kultivieren, das die interessierenden Substanzen enthält. Man kann aber auch in einfacher Weise die Tiere in den zu untersuchenden Materialien baden. Diese Variante ist nicht nur besonders einfach, sondern ermöglicht auch eine leichtere Standardisierung der Konzentrationen. Für eine Untersuchung werden gut ernährte Populationen der Tiere herangezogen, die dann mit unterschiedlichen Lösungen der Materialien und in unterschiedlichen Konzentrationen inkubiert werden. Die Versuche der Erfinder haben nun gezeigt, daß Substanzen, die sowohl schmerzauslösend als auch schmerzmildernd wirken, das Reflexverhalten des Nematoden deutlich beeinflussen. So läßt sich einerseits mit einer Hyperalgesie-auslösenden Substanz, wie Capsaicin, als auch mit einem wirksamen und spezifischen Inhibitor für Capsaicin, nämlich Capzazepin, das Reflexverhalten beeinflussen. Diese Beobachtung, nämlich die Modifizierung des Reflexverhaltens mit von Säugetieren bekannten Agonisten/Antagonisten-Paaren, erlaubt einen Rückschluß auf eine mögliche Wirksamkeit eines Materials, dessen Eignung als Pharmakon zu untersuchen ist.

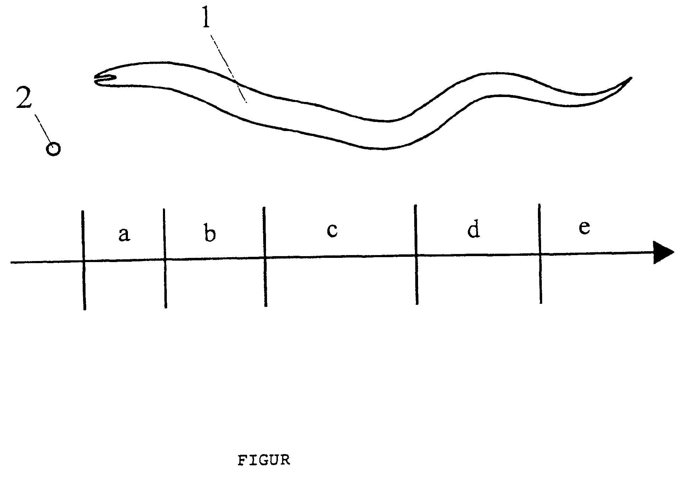

Die Figur zeigt schematisch einen Wurm (1) mit einer Länge von etwa 1,3 mm und einem Durchmesser 80 µm. Der Wurm (1) kann dann mit einem focussierten Laserstrahl belichtet werden, der eine lokale Erwärmung mit sich bringt. Der Laserspot (2) hat einen Durchmesser von etwa 30 µm und wird im erfindungsgemäßen Verfahren vorzugsweise auf die Kopf- bzw. Schwanzbereiche des Wurmes gelenkt. Der Kopfbereich entspricht in etwa den Bereichen a und b nach der schematischen Zeichnung, der Schwanzbereich ist der Bereich e. Regelmäßig beobachtet man bei *C. elegans* vom Wildtyp das hier diskutierte Reflexverhalten in den verschiedenen Bereichen mit folgender Häufigkeit: a - 98%, b - 71%, c - 0%, d - 0%, e - 49%.

Die Untersuchungen der Erfinder haben somit gezeigt, daß die Verwendung von *C. elegans* als Tiermodell zur Untersuchung von Materialien hinsichtlich ihrer Eignung als Pharmakon beim Menschen oder anderen Säugetieren zuverlässige Voraussagen gestattet, insbesondere wenn es um die Empfindung bzw. Wahrnehmung von Schmerzen geht, und zwar sowohl hinsichtlich algetischer oder hyperalgetischer als auch analgetischer Wirkungen. Es hat sich gezeigt, daß ein Ansprechen der sogenannten Nozizeptoren bei *C. elegans* in einfacher Weise mit einer erwärmten Drahtspitze (z.B. Mikrolötpistole, erhitzter Spatel etc.) oder mit einem focussierten Laserstrahl erreicht werden kann. Ein normaler Reflex auf einen thermischen Reiz von beispielsweise 32-40°C im Kopfbereich des Nematoden läßt sich wie folgt beschreiben:
I) Schneller reflektiver Rückzug um mindestens eine volle Körperlänge und anschließende Ausrichtung des Kopfes in eine andere Richtung.
II) Schneller reflektiver Rückzug, aber ohne Rückwärtsbewegung.
III) Langsamer Rückzug.

Es konnte beobachtet werden, daß regelmäßig mindestens 80% der Tiere normalerweise ein Verhalten gemäß I) zeigten.

Zur Standardisierung des erfindungsgemäßen Verfahrens ist es ratsam, einen Laser einzusetzen, da mit diesem in wiederholbarer Weise ein gleichbleibender thermischer Reiz auf die Nematoden aufgebracht werden kann. Da der Laserstrahl auch keine permanente Schädigung der Nematoden herbeiführt, läßt sich das erfindungsgemäße Verfahren wiederholt durchführen.

Die Erfindung wird in den folgenden Beispielen erläutert.

### Beispiele

Die gut ernährten (etwa 100; etwa 1,3 mm lang; Durchmesser 80 µm) Testtiere (*C. elegans*) wurden in den Untersuchungen mit einem Infrarotlaser (685 ± 0,5 nm) bestrahlt. Der Laserstrahl wurde jeweils auf einen Fleck im Kopfbereich des Tieres mit einem Durchmesser von 30µm fokussiert, wobei auf einer Agar-Fläche eine lokale Erwärmung auf 33,5 ± 1°C festgestellt wurde. Um das Verhalten der Tiere näher zu untersuchen, wurden diese einem nozizeptiven Reiz ausgesetzt, nach dem die Tiere jeweils in unterschiedlichen Konzentrationen zwischen 1 und 100µm Capsaicin inkubiert wurden. Es wurde eine starke Reaktion auch noch nach 1 h im Anschluß an die Inkubation festgestellt. Diese Reaktion zeigt sich darin, daß über 90% der Tiere einen Reflex gemäß I) zeigten, wobei in der Kontrollgruppe nur 80% der Tiere diesen Reflex aufwiesen. Noch deutlicher konnte man diese Reaktion bei Mutanten beobachten, deren Normalverhalten darin besteht, daß lediglich 70% der Tiere gemäß I) reagieren.

Diese Beispiele zeigen, daß man mit Substanzen, die beim Menschen eine hyperalgetische Wirkung zeigen, den hier interessierenden Reflex verstärken kann. Ein umgekehrtes, d.h. abgeschwächtes Reflexverhalten beobachtet man bei Substanzen, die schmerzlindernd sein können.

## Patentansprüche

1. Verfahren zur Untersuchung der Eignung eines Materials als Pharmakon betreffend die Schmerzempfindung, **dadurch gekennzeichnet daß**
1. zumindest ein Nematode eingesetzt wird, der dem Material ausgesetzt wurde,
2. der Nematode örtlich einem thermischen Reiz ausgesetzt wird und
3. eine Reaktion bei Abweichung von einem Normalverhalten aufgezeichnet wird.

2. Verfahren nach Anspruch 1, **dadurch gekennzeichnet, daß** der Nematode dem Stamm Nematoda angehört.

3. Verfahren nach Anspruch 2, **dadurch gekennzeichnet, daß** der Nematode der Gattung Caenorhabditis angehört.

4. Verfahren nach Anspruch 1, **dadurch gekennzeichnet, daß** die Schmerzempfindung schmerzmindernd oder schmerzstillend ist.

5. Verfahren nach einem der vorstehenden Ansprüche, **dadurch gekennzeichnet, daß** der Nematode in seinem Kopf- oder Schwanzbereich dem thermischen Reiz ausgesetzt wird.

6. Verfahren nach Anspruch 5, **dadurch gekennzeichnet, daß** der thermische Reiz außerhalb eines Temperaturbereichs von 13-26°C liegt.

7. Verfahren nach Anspruch 6, **dadurch gekennzeichnet, daß** der thermische Reiz mindestens eine Temperatur von 28°C an der Stelle, auf der der Nematode dem thermischen Reiz ausgesetzt wird, ausmacht.

8. Verfahren nach einem der vorstehenden Ansprüche, **dadurch gekennzeichnet, daß** der thermische Reiz örtlich auf einen Bereich von-10-100 µm² begrenzt wird.

9. Verfahren nach einem der vorstehenden Ansprüche, **dadurch gekennzeichnet, dass** der Reiz mittels eines Lasers oder einer erwärmten Drahtspitze ausgelöst wird.

## Revendications

1. Procédé pour l'examen de la qualification d'une substance en tant qu'agent pharmaceutique concernant la sensation de douleur, **caractérisé en ce que**
1. on utilise au moins un nématode qui a été exposé à la substance,
2. le nématode est exposé localement à un stimulus thermique et
3. on enregistre une réaction dans le cas d'un écart par rapport à un comportement normal.

2. Procédé selon la revendication 1, **caractérisé en ce que** le nématode appartient à l'embranchement *Nematoda.*

3. Procédé selon la revendication 2, **caractérisé en ce que** le nématode appartient au genre *Caenorhabditis*.

4. Procédé selon la revendication 1, **caractérisé en ce que** la sensation de douleur est réduite ou supprimée.

5. Procédé selon l'une des revendications précédentes, **caractérisé en ce que** le nématode est exposé au stimulus thermique dans la région de sa tête ou de sa queue.

6. Procédé selon la revendication 5, **caractérisé en ce que** le stimulus thermique se situe hors d'une plage de température de 13-26°C.

7. Procédé selon la revendication 6, **caractérisé en ce que** le stimulus thermique représente au moins une température de 28°C au point où le nématode est exposé au stimulus thermique.

8. Procédé selon l'une des revendications précédentes, **caractérisé en ce que** le stimulus thermique est limité localement à une zone de 10-100 µm².

9. Procédé selon l'une des revendications précédentes, **caractérisé en ce que** le stimulus est déclenché au moyen d'un laser ou d'une pointe chauffée d'un fil métallique.

## Claims

1. Method of investigating the suitability of a material as a medicament concerned with the sensation of pain, wherein
1. at least one nematode is used which has been exposed to the material,
2. the nematode is exposed to a local thermal stimulus, and
3. a reaction is recorded if it deviates from normal behavior.

2. Method according to claim 1, wherein the nematode belongs to the strain Nematode.

3. Method according to claim 2, wherein the nematode belongs to the genus Caenorhabditis.

4. Method according to claim 1, wherein the sensation of pain is reduced or relieved.

5. Method according to any of the preceding claims, wherein the nematode is exposed to the thermal stimulus in its head or tail region.

6. Method according to claim 5, wherein the thermal stimulus is outside the temperature range 13-26°C

7. Method according to claim 6, wherein the thermal stimulus produces a temperature of at least 28°C at the site at which the nematode is exposed to the thermal stimulus.

8. Method according to any of the preceding claims, wherein the thermal stimulus is limited to a local area of 10-100 µm².

9. Method according to any of the preceding claims, wherein the stimulus is produced by means of a laser or a heated wire tip.
